# EUROPÄISCHE PATENTANMELDUNG

(11) **EP 4 345 533 A1**
(43) Veröffentlichungstag der Anmeldung: **03.04.2024**
(21) Anmeldenummer: 22198829.8
(22) Anmeldetag: 29.09.2022
(51) Int. Cl.: G02C 7/10, G02F 1/1333, A61F 9/02

(54) **BRILLENVORRICHTUNG, BRILLE, VERFAHREN ZU EINEM BETRIEB**

(71) Anmelder: Optrel Holding AG, 9630 Wattwil (CH)
(72) Erfinder: BLÖCHLINGER, Daniel, 8735 St. Gallenkappel (CH); KOCH, Marco, 9613 Mühlrüti (CH)
(74) Vertreter: Daub, Thomas

(57) **Zusammenfassung**

Die Erfindung geht aus von Brillenvorrichtung (10), insbesondere Skibrille, Sonnenbrille, Motorradbrille oder dergleichen, mit einer Durchsichteinheit (12), welche zumindest ein Verdunkelungselement (14), insbesondere eine Flüssigkristallzelle, aufweist, das zu einer Verdunkelung zumindest eines Teilbereichs der Durchsichteinheit (12) vorgesehen ist, und mit einer Lichtsensoreinheit (16), welche dazu vorgesehen ist, ein von einer Umgebungshelligkeit abhängiges Sensorsignal zu erzeugen, das Basis für einen, insbesondere automatisch eingestellten, Grad einer, insbesondere variablen, Verdunkelung des Verdunkelungselements (14) ist.

Es wird eine Brillenvorrichtung (10) mit einer Abschalteinheit (18) vorgeschlagen, welche zumindest eine, insbesondere manuell bedienbare, Abschaltfunktion für das Sensorsignal der Lichtsensoreinheit (16) bereitstellt.

## Beschreibung

### Stand der Technik

Die Erfindung betrifft eine Brillenvorrichtung nach dem Oberbegriff des Anspruchs 1, eine Brille nach dem Anspruch 9 und ein Verfahren nach dem Oberbegriff des Anspruchs 10.

Es ist bereits eine Brillenvorrichtung mit einer Durchsichteinheit, welche zumindest ein Verdunkelungselement aufweist, das zu einer Verdunkelung zumindest eines Teilbereichs der Durchsichteinheit vorgesehen ist, und mit einer Lichtsensoreinheit, welche dazu vorgesehen ist, ein von einer Umgebungshelligkeit abhängiges Sensorsignal zu erzeugen, das Basis für einen Grad einer Verdunkelung des Verdunkelungselements ist, vorgeschlagen worden.

Die Aufgabe der Erfindung besteht insbesondere darin, eine gattungsgemäße Vorrichtung mit verbesserten Eigenschaften hinsichtlich einer Funktion bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Brillenvorrichtung, insbesondere einer Skibrille, einer Sonnenbrille, einer Motorradbrille oder dergleichen, mit einer Durchsichteinheit, welche zumindest ein Verdunkelungselement, insbesondere eine Flüssigkristallzelle, aufweist, das zu einer Verdunkelung zumindest eines Teilbereichs der Durchsichteinheit vorgesehen ist, und mit einer Lichtsensoreinheit, welche dazu vorgesehen ist, ein von einer Umgebungshelligkeit abhängiges Sensorsignal zu erzeugen, das Basis für einen, insbesondere automatisch eingestellten, Grad einer, insbesondere variablen, Verdunkelung des Verdunkelungselements ist.

Es wird vorgeschlagen, dass die Brillenvorrichtung eine Abschalteinheit aufweist, welche zumindest eine, insbesondere manuell bedienbare, Abschaltfunktion für das Sensorsignal der Lichtsensoreinheit bereitstellt. Durch die erfindungsgemäße Ausgestaltung der Brillenvorrichtung kann vorteilhaft ein Komfort verbessert werden, da insbesondere der Grad der Verdunkelung von dem Verdunkelungselement für jeden Benutzer individuell einstellbar ist. Vorteilhaft kann eine Sicherheit erhöht werden, da insbesondere bei Bedarf durch den Benutzer in jeder Betriebssituation, wie beispielsweise Umgebungsbedingungen mit Nebel oder Regen, die Verdunkelung abgeschaltet werden kann und somit zu jeder Zeit eine gute Sicht gewährleistet werden kann.

Vorzugsweise ist die Brillenvorrichtung als eine Skibrille, Sonnenbrille, Motorradbrille oder eine vergleichbare Brille, insbesondere Freizeitbrille, ausgebildet. Dabei kann die Brillenvorrichtung Teil einer Brille und/oder eines Helmes und/oder eines Schirms und/oder einer Maske und/oder in einer vergleichbaren Anwendung, welche einen Benutzer vor Helligkeit, insbesondere UV-Strahlung, schützt, sein. Vorzugsweise ist die Durchsichteinheit als eine Scheibe und/oder ein Brillenglas und/oder als ein Visier ausgebildet. Vorzugsweise ist die Durchsichteinheit zumindest aus einem, insbesondere bruchsicheren, Kunststoff und/oder einem Verbundglas und/oder aus einem handelsüblichen Glaswerkstoff oder aus einem vergleichbaren durchsichtigen Werkstoff ausgebildet. Vorzugsweise ist die Durchsichteinheit aus mehreren, insbesondere gleich großen und deckungsgleich angeordneten, Scheiben ausgebildet. Vorzugsweise sind alle, eine Durchsichteinheit bildenden Scheiben fest, insbesondere form- und/oder stoffschlüssig, miteinander verbunden. Vorzugsweise ist die Durchsichteinheit transparent und/oder teiltransparent ausgebildet. Vorzugsweise ist die transparente und/oder teiltransparente Durchsichteinheit farbig, beschichtet und/oder bedampft und/oder getönt ausgebildet. Insbesondere kann die Durchsichteinheit eine photochromatische Schicht aufweisen. Die photochromatische Schicht ist dazu vorgesehen, unter Einwirkung von elektromagnetischer Strahlung, insbesondere UV-Strahlung, eine spektrale Transmission im sichtbaren Bereich, reversibel, insbesondere so lange die photochromatische Schicht der elektromagnetischen Strahlung ausgesetzt ist, zu ändern. Vorzugsweise weist die Durchsichteinheit zumindest ein farbig und transparent ausgebildetes Element, insbesondere Durchsichtelement, auf. Vorzugsweise ist die, insbesondere farbige Tönung der Durchsichteinheit dazu vorgesehen, UV-Strahlen vom menschlichen Auge des Benutzers fernzuhalten und/oder ein Blenden durch lokal auftretende gebündelte Sonnenstrahlung zu verhindern. Vorzugsweise weist die Durchsichteinheit zumindest ein Verdunkelungselement auf. Vorzugsweise ist das Verdunkelungselement als eine Flüssigkeitskristallzelle, insbesondere LCD-Scheibe (Liquid Crystal Display), ausgebildet. Vorzugsweise weist die Flüssigkristallzelle eine spannungsabhängige, insbesondere stufenlose und/oder variable, Lichtdurchlässigkeit auf. Vorzugsweise ist die Lichtdurchlässigkeit von der Flüssigkeitskristallzelle über eine Spannung steuerbar. Vorzugsweise ist das Verdunkelungselement dazu vorgesehen, eine Helligkeit, insbesondere Umgebungshelligkeit, für das menschliche Auge von einem Benutzer zu reduzieren und das menschliche Auge und/oder die menschliche Haut von dem Benutzer vor UV-Strahlung zu schützen. Insbesondere ist das Verdunkelungselement dazu vorgesehen, eine Transparenz der Durchsichteinheit zu variieren, insbesondere zu erhöhen oder zu senken. Vorzugsweise weist die Durchsichteinheit und/oder das Verdunkelungselement aus einer Betrachtungsrichtung des die Brille tragenden Benutzers einen, insbesondere konkav, gebogenen Verlauf auf.

Unter einer "Umgebungshelligkeit" soll dabei insbesondere eine Helligkeit der Umwelt, insbesondere eine Helligkeit außerhalb eines Brillenraums, welcher das Sichtfeld eines menschlichen Auges durch die Brille mit der Brillenvorrichtung begrenzt, verstanden werden.

Vorzugsweise ist die Lichtsensoreinheit als eine Fotodiode oder eine Solarzelle, insbesondere photovoltaische Zelle, oder als ein vergleichbares elektrisches Halbleiterbauelement, welches Sonnenlicht, insbesondere sichtbare elektromagnetische Strahlung, in elektrische Energie, insbesondere eine elektrische Spannung, umwandelt, ausgebildet. Vorzugsweise ist die Lichtsensoreinheit in die Brille, insbesondere einen Rahmen und/oder einen Bügel der Brille, integriert oder an der Brille angeordnet. Vorzugsweise weist die Lichtsensoreinheit zumindest ein Sensorfeld zu einer Detektion von der Umgebungshelligkeit auf. Das Sensorfeld könnte eine Beschichtung, wie beispielsweise eine photochromatische Schicht, aufweisen. Insbesondere ist die photochromatische Schicht dazu vorgesehen, eine auf das Sensorfeld einstrahlende Helligkeit zu reduzieren, wobei die photochromatische Schicht den Grad der Verdunkelung von dem Verdunkelungselement beeinflusst. Vorzugsweise weist die Lichtsensoreinheit eine Platine als Trägerelement auf. Vorzugsweise weist die Lichtsensoreinheit eine Steuer- und Regeleinheit auf, welche dazu vorgesehen ist, ein von dem Sensorfeld erzeugtes Sensorsignal, insbesondere elektrisches Spannungssignal, zu verarbeiten. Unter einer "Steuer- und/oder Regeleinheit" soll insbesondere eine Einheit mit zumindest einer Steuerelektronik verstanden werden. Unter einer "Steuerelektronik" soll insbesondere eine Einheit mit einem Prozessor und mit einem Speicher sowie mit einem in dem Speicher gespeicherten Betriebsprogramm verstanden werden. Vorzugsweise ist das Sensorsignal als eine elektrische Spannung ausgebildet. Vorzugsweise ist das Sensorsignal, insbesondere zumindest im Wesentlichen linear, abhängig von der Helligkeit, insbesondere einer Lichtintensität der elektromagnetischen Strahlung. Vorzugsweise ist das Sensorsignal dazu vorgesehen, den Grad der Verdunkelung von dem Verdunkelungselement einzustellen, insbesondere zu steuern. Vorzugsweise dient das Sensorsignal als elektrische Energiequelle zu der Verdunkelung von dem Verdunkelungselement und/oder zu einer elektrischen Versorgung von der Brillenvorrichtung, insbesondere einer elektrischen Versorgung eines Energiespeichers, welcher die Brillenvorrichtung mit elektrischer Energie versorgt.

Vorzugsweise ist die Abschalteinheit dazu vorgesehen, eine von dem Verdunkelungselement erzeugte (ggf. zu einer Tönung zusätzliche) Verdunkelung vollständig abzuschalten und/oder zu verhindern. Vorzugsweise weist die Abschalteinheit eine Abschaltfunktion auf, welche das Sensorsignal, insbesondere die elektrische Spannung von der Steuereinheit und/oder der elektrischen Zuleitung von der Verdunkelungseinheit trennt. Vorzugsweise ist die Abschaltfunktion von der Abschalteinheit dazu vorgesehen, eine Umgebungshelligkeit zumindest im Wesentlichen zu eliminieren und/oder ein Spannungssignal, insbesondere die Erzeugung der elektrischen Spannung, der Sensoreinheit, zu unterdrücken. Vorzugsweise ist die Abschaltfunktion von der Abschalteinheit manuell, insbesondere durch den Benutzer von der Brillenvorrichtung oder von einem Dritten, bedienbar.

Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Ferner wird vorgeschlagen, dass die Abschalteinheit ein mechanisches Abdeckelement aufweist, welches in zumindest einem Betriebszustand dazu vorgesehen ist, zumindest alle Messbereiche der Lichtsensoreinheit zumindest im Wesentlichen vollständig abzudecken. Vorteilhaft kann die Abschalteinheit besonders einfach betätigt werden, da insbesondere die Abschalteinheit auch mit Handschuhen oder mit einem Arm einfach betätigt werden kann. Vorzugsweise ist das mechanische Abdeckelement an einem Rahmen und/oder Bügel von der Brille angeordnet. Vorzugsweise ist das mechanische Abdeckelement entlang des Rahmens und/oder des Bügels von der Brille beweglich ausgebildet/ gelagert angeordnet. Vorzugsweise ist das mechanische Abdeckelement als ein, insbesondere verschiebbarer und/oder einstellbarer, Schieber oder Riegel oder Klappe ausgebildet. Es ist auch denkbar, dass das mechanische Abdeckelement rotatorisch öffen- oder schließbar ausgebildet ist. Vorzugsweise ist das mechanische Abdeckelement opak, insbesondere lichtundurchlässig, ausgebildet. Vorzugsweise ist das mechanische Abdeckelement dazu vorgesehen, elektromagnetische Strahlung, insbesondere mit einem Wellenlängenbereich von sichtbarem Licht, von den Messbereichen der Lichtsensoreinheit abzuschirmen. Vorzugsweise schirmt das Abdeckelement einen Teilbereich von der Lichtsensoreinheit gegen elektromagnetische Strahlung ab, welcher nicht durch weitere Bauteile der Brille, insbesondere den die Lichtsensoreinheit befestigenden Rahmen und/oder Bügel von der Brille abgedeckt ist. Vorzugsweise ist das mechanische Abdeckelement und/oder der Rahmen und/oder der Bügel von der Brille, aus einem, insbesondere durch eine Herstellung in einem Ein- oder Mehrkomponentenspritzverfahren gefertigten, Kunststoff ausgebildet. Unter einer "im Wesentlichen vollständigen" Abdeckung soll insbesondere eine Abdeckung von mehr als 90%, vorzugsweise von mehr als 95% und bevorzugt von mehr als 99% verstanden werden. Besonders bevorzugt deckt das mechanische Abdeckelement in dem Betriebszustand die Messbereiche vollständig (zu 100%) ab.

Des Weiteren wird vorgeschlagen, dass das mechanische Abdeckelement in zumindest einem weiteren Betriebszustand dazu vorgesehen ist, die Messbereiche der Lichtsensoreinheit lediglich teilweise abzudecken. Vorteilhaft kann ein Komfort für den Benutzer der Brille mit der Brillenvorrichtung verbessert werden, da insbesondere die Helligkeit von dem Verdunkelungselement, insbesondere der Scheibe, individuell auf die Anforderungen des Benutzers angepasst werden kann. Vorzugsweise ist eine Bewegung von dem mechanischen Abdeckelement, insbesondere zu einem Abdecken von dem Messbereich der Lichtsensoreinheit, stufenlos ausgebildet. Vorzugsweise ist jede beliebige Position des mechanischen Abdeckelements kraftschlüssig mit dem Rahmen und/oder dem Bügel von der Brille befestigt und/oder fixiert.

Zusätzlich wird vorgeschlagen, dass das mechanische Abdeckelement zumindest eine, vorzugsweise vollständig in Umfangsrichtung von dem Abdeckelement umrandete, Durchgangsöffnung aufweist, welche dazu vorgesehen ist, in zumindest einem (zusätzlichen) weiteren Betriebszustand vollständig mit zumindest einem Messbereich der Lichtsensoreinheit zu überlappen. Vorteilhaft kann der Messbereich von der Lichtsensoreinheit vollständig der Umgebungshelligkeit ausgesetzt werden, wodurch insbesondere eine maximale Verdunkelung von dem Verdunkelungselement, bei gegebener Umgebungshelligkeit erreicht werden kann. Vorzugsweise ist die Durchgangsöffnung größer als und/oder zumindest im Wesentlichen gleich groß wie der Messbereich von der Lichtsensoreinheit ausgebildet. Vorzugsweise ist die Durchgangsöffnung rechteckig, insbesondere quadratisch, ausgebildet. Es ist auch denkbar, dass die Durchgangsöffnung mehreckig oder rund ausgebildet ist. Insbesondere ist die Durchgangsöffnung des mechanischen Abdeckelements größer oder zumindest gleich groß wie der korrespondierende Messbereich der Lichtsensoreinheit.

Außerdem wird vorgeschlagen, dass die Durchgangsöffnung des mechanischen Abdeckelements in dem Betriebszustand, in dem insbesondere alle Messbereiche der Lichtsensoreinheit zumindest im Wesentlichen vollständig abgedeckt sind, überlappungsfrei mit dem Messbereich, und insbesondere mit allen weiteren Messbereichen der Lichtsensoreinheit, ist. Vorteilhaft kann eine Sicherheit von dem Benutzer erhöht werden, da insbesondere bei Bedarf durch den Benutzer in jeder Betriebssituation, wie beispielsweise Umgebungsbedingungen mit Nebel oder Regen, die Verdunkelung mechanisch, insbesondere auch in einem Betriebszustand ohne elektrische Energie, beispielsweise bei vollständig entladenem Akku, abgeschaltet werden kann und somit zu jeder Zeit eine gute Sicht gewährleistet werden kann. Vorzugsweise weist das mechanische Abdeckelement zumindest einen opaken und/oder teilweise opaken Teilbereich auf, welche größer ist als der Messbereich der Lichtsensoreinheit. Vorzugsweise ist das mechanische Abdeckelement, insbesondere in dem Betriebszustand mit Durchgangsöffnungen, welche überlappungsfrei mit den Messbereichen angeordnet sind, dazu vorgesehen, die Umgebungshelligkeit, insbesondere vollständig, von dem Messbereich abzuschirmen und/oder eine Erzeugung des Spannungssignals von der Lichtsensoreinheit zu unterdrücken.

Weiterhin wird vorgeschlagen, dass die Lichtsensoreinheit zumindest eine Solarzelle und/oder zumindest eine Fotodiode aufweist. Vorteilhaft kann ein Bedienkomfort verbessert werden, da insbesondere durch die Fotodiode ein Grad der Dunkelheit automatisch, in Abhängigkeit von der Umgebungshelligkeit, eingestellt werden kann. Vorteilhaft kann ein Wartungsintervall verlängert oder vollständig auf ein Wartungsintervall verzichtet werden, da insbesondere durch die Solarzelle zu jeder Zeit eine Spannungsversorgung gewährleistet ist und daher auf eine, insbesondere wartungsintensive externe Spannungsversorgung, wie beispielsweise einen Akku, verzichtet werden kann. Vorzugsweise ist die Fotodiode dazu vorgesehen, eine Helligkeit, insbesondere mit hoher Empfindlichkeit, zu detektieren. Vorzugsweise ist eine Solarzelle dazu vorgesehen, elektrischen Strom, insbesondere zu einer elektrischen Versorgung der Brillenvorrichtung, zu erzeugen.

Ergänzend wird vorgeschlagen, dass das mechanische Abdeckelement an dem Rahmen und/oder an einem Brillenbügel, insbesondere integriert oder montiert, angeordnet ist. Vorteilhaft kann ein Komfort verbessert werden, da insbesondere das mechanische Abdeckelement an einer für den Benutzer gut zugänglichen Position angeordnet ist. Vorteilhaft kann das mechanische Abdeckelement nachgerüstet werden, da insbesondere eine nachträgliche Befestigung von dem Abdeckelement und/oder ein nachträglicher Einbau von der Lichtsensoreinheit und/oder des Verdunkelungselements möglich ist. Vorzugsweise ist das mechanische Abdeckelement formschlüssig an dem Rahmen und/oder dem Bügel der Brille angeordnet. Vorzugsweise ist das mechanische Abdeckelement oberhalb des Verdunkelungselements angeordnet. Vorzugsweise ist das mechanische Abdeckelement außerhalb eines Sichtfelds von dem Benutzer angeordnet. Vorzugsweise ist das mechanische Abdeckelement entlang einer Längserstreckung von dem Verdunkelungselement, insbesondere zumindest abschnittsweise rotationsbeweglich um einen Rotationsmittelpunkt von dem, insbesondere konkav, gebogenen Verlauf der Durchsichteinheit, und/oder des Verdunkelungselements, beweglich gelagert.

Ferner wird vorgeschlagen, dass die Abschalteinheit, insbesondere alternativ oder zusätzlich zu dem manuell beweglichen mechanischen Abdeckelement, mittels einer Fernsteuerung, insbesondere einem Mobilgerät, wie z.B. einem Smartphone, bedienbar ist. Vorteilhaft kann eine manuelle Steuerung automatisiert werden, da insbesondere eine Fernsteuerung über ein Smartphone auch eine tageszeit- und/oder ortsabhängige Steuerung ermöglicht. Vorteilhaft kann die Abschalteinheit auch von einem Dritten bedient werden, da insbesondere eine Bedienung aus größerer Entfernung möglich ist. Vorzugsweise ist die Fernsteuerung zusätzlich zu der mechanischen Abdeckeinheit von der Abschalteinheit umfasst. Vorzugsweise ist die Fernsteuerung zu einer An- und Abschaltung und/oder zu einer Regelung von dem Grad der Dunkelheit von der Durchsichteinheit vorgesehen.

Zusätzlich wird eine Brille mit der Brillenvorrichtung vorgeschlagen. Vorteilhaft ist ein Bedienkomfort verbessert, da insbesondere die Brille mit der Brillenvorrichtung den Grad der Dunkelheit von der Durchsichteinheit automatisch anpasst, wenn sich die Umgebungshelligkeit verändert.

Des Weiteren wird ein Verfahren zu einem Betrieb der Brillenvorrichtung vorgeschlagen, wobei in zumindest einem Abschaltschritt das Sensorsignal über eine, insbesondere manuell bedienbare, Abschalteinheit abgeschaltet wird. Vorteilhaft kann der Benutzer selbst entscheiden, ob die Durchsichteinheit durch das Verdunkelungselement verdunkelt werden soll.

Ergänzend wird vorgeschlagen, dass in dem Abschaltschritt das Sensorsignal durch ein mechanisches Abdecken einer das Sensorsignal erzeugenden Lichtsensoreinheit abgeschaltet wird. Vorteilhaft kann der Benutzer selbst entscheiden, ob und/oder wie stark die Durchsichteinheit durch das Verdunkelungselement verdunkelt werden soll. Vorteilhaft wird die Brille individuell auf die Bedürfnisse des Bedieners angepasst, da insbesondere ein Grad der Dunkelheit durch die stufenlose Verstellung von dem mechanischen Abschaltelement bestimmt werden kann.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### Es zeigen:

- Fig. 1: eine schematische Explosionsdarstellung einer Brille mit einer Brillenvorrichtung,
- Fig. 2: eine teilweise wieder zusammengefügte schematische Explosionsdarstellung der Brille mit der Brillenvorrichtung,
- Fig. 3a: eine schematische Darstellung eines Ausschnitts der Brille mit der Brillenvorrichtung mit einem mechanischen Abdeckelement in einem Betriebszustand,
- Fig. 3b: eine schematische Darstellung des Ausschnitts der Brille mit der Brillenvorrichtung mit dem mechanischen Abdeckelement in einem weiteren Betriebszustand,
- Fig. 3c: eine schematische Darstellung des Ausschnitts der Brille mit der Brillenvorrichtung mit dem mechanischen Abdeckelement in einem zusätzlichen weiteren Betriebszustand und
- Fig. 4: ein schematisches Ablaufdiagramm eines Verfahrens zu einem Betrieb der Brillenvorrichtung.

### Beschreibung der Ausführungsbeispiele

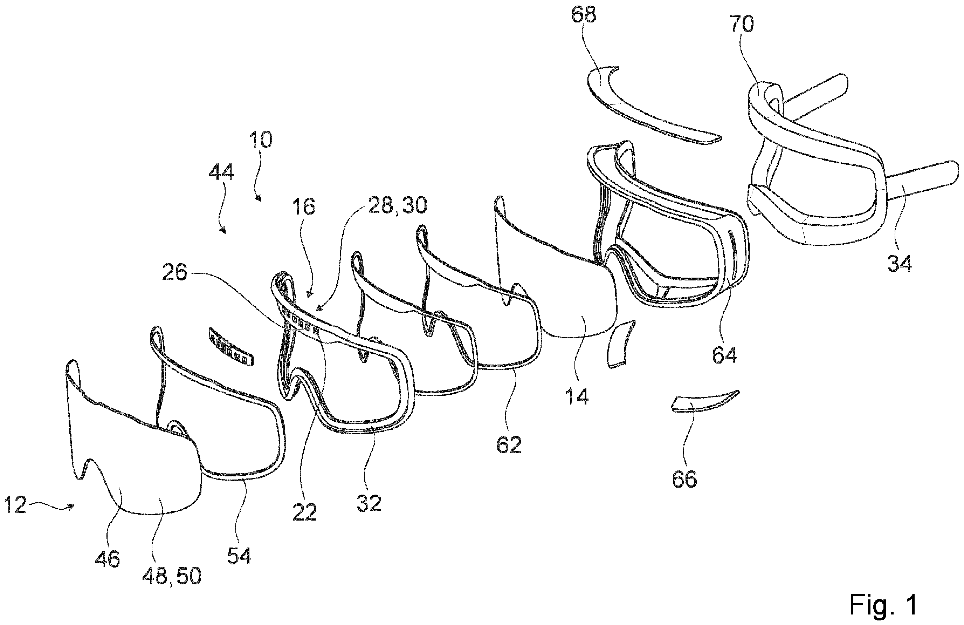

Die Figur 1 zeigt eine Brille 44. Die Brille 44 weist eine Brillenvorrichtung 10 auf. Die Brillenvorrichtung 10 ist als eine Skibrille ausgebildet. Die Brillenvorrichtung 10 könnte auch als eine Sonnenbrille oder eine Motorradbrille oder eine vergleichbare Brille als Komfortbrille für eine Freizeitanwendung oder eine Arbeitsschutzbrille für eine gewerbliche Anwendung ausgebildet sein. Die Brillenvorrichtung 10 weist eine Durchsichteinheit 12 auf. Die Durchsichteinheit 12 ist als eine Scheibe 48 ausgebildet. Die Scheibe 48 der Durchsichteinheit 12 ist aus einem Kunststoff ausgebildet. Alternativ könnte die Scheibe 48 aus einem Verbundglas oder einem Glaswerkstoff ausgebildet sein. Die Scheibe 48 ist transparent ausgebildet. Alternativ könnte die Scheibe 48 teiltransparent ausgebildet sein. Alternativ könnte die Scheibe 48 abschnittsweise transparent ausgebildet sein. Die Scheibe 48 der Durchsichteinheit 12 ist getönt ausgebildet. Alternativ könnte die Scheibe 48 der Durchsichteinheit 12 farbig und/oder beschichtet und/oder bedampft ausgebildet sein. Die Scheibe 48 weist einen Teilbereich 46 auf. Der Teilbereich 46 erstreckt sich über den gesamten transparenten Bereich der Durchsichteinheit 12. Alternativ könnte sich der Teilbereich 46 nur teilweise über den transparenten Bereich der Durchsichteinheit 12 erstrecken. Die Durchsichteinheit 12 weist ein Verdunkelungselement 14 auf. Das Verdunkelungselement 14 ist als eine Flüssigkristallzelle ausgebildet. Das Verdunkelungselement 14 ist zu einer Verdunkelung von dem Teilbereich 46 der Durchsichteinheit 12 vorgesehen. Das Verdunkelungselement 14 ist elektrisch ansteuerbar. Das Verdunkelungselement 14 ist stufenlos verdunkelbar ausgebildet. Die Durchsichteinheit 12 könnte weitere Scheiben 50 aufweisen. Die weitere Scheibe 50 könnte als eine Anti-Fog-Scheibe und/oder eine Antikratz-Scheibe und/oder eine Heads-Up-Display-Scheibe und/oder eine Sehstärke-Scheibe oder eine vergleichbare Scheibe ausgebildet sein. Die Scheibe 48 und das Verdunkelungselement 14 der Durchsichteinheit 12 und jede weitere Scheibe 50 sind gleich groß ausgebildet. Die Scheibe 48 und das Verdunkelungselement 14 sind deckungsgleich zueinander angeordnet. Die Scheibe 48 und das Verdunkelungselement 14 sind stoffschlüssig miteinander verbunden. Die Scheibe 48 und das Verdunkelungselement 14 könnten alternativ auch formschlüssig miteinander verbunden sein. Jede weitere Scheibe 50 ist stoffschlüssig und/oder formschlüssig mit der Durchsichteinheit 12 und/oder der Scheibe 48 und/oder dem Verdunkelungselement 14 verbunden.

Die Brillenvorrichtung 10 weist einen Rahmen 32 auf. Der Rahmen 32 umgibt die Durchsichteinheit 12. Der Rahmen 32 befestigt die Durchsichteinheit 12. Der Rahmen 32 umgreift die Durchsichteinheit 12 vollständig. Alternativ könnte der Rahmen 32 die Durchsichteinheit 12 nur teilweise umgreifen. Die Brillenvorrichtung 10 weist ein Klebeelement 54 und ein weiteres Klebeelement 62 auf. Das Klebeelement 54 und/oder das weitere Klebeelement 62 ist dazu vorgesehen, die Durchsichteinheit 12 an dem Rahmen 32 zu fixieren. Der Rahmen 32 ist aus einem Kunststoff hergestellt. Der Rahmen 32 begrenzt ein Sichtfeld eines Benutzers. Die Brillenvorrichtung 10 weist einen Brillenbügel 34 auf. Der Brillenbügel 34 ist an dem Rahmen 32 fixiert. Alternativ könnte der Brillenbügel 34 einstückig mit dem Rahmen 32 ausgebildet sein. Alternativ könnte der Brillenbügel 34 auch als ein Brillenband ausgebildet sein. Alternativ könnte die Brillenvorrichtung 10 an einem Helm oder einem Visier oder vergleichbarer Vorrichtung angeordnet sein, welche die Funktion des Brillenbügels 34 übernimmt. Die Brillenvorrichtung 10 weist einen weiteren Rahmen 64 auf. Der weitere Rahmen 64 ist aus einem weichen und/oder luftdurchlässigen Werkstoff, wie beispielsweise einem flexiblen Kunststoff oder einem Schaumstoff, ausgebildet. Der weitere Rahmen 64 weist Lüftungsöffnungen auf. Der weitere Rahmen 64 ist dazu vorgesehen, eine Belüftung zu ermöglichen und dadurch ein Beschlagen der Durchsichteinheit 12 zu verhindern. Die Brillenvorrichtung 10 weist ein Abdeckelement 66 und/oder ein weiteres Abdeckelement 68 auf. Das Abdeckelement 66 und das weitere Abdeckelement 68 sind aus einem luftdurchlässigen Werkstoff, wie beispielsweise einem Schaumstoff, ausgebildet. Das Abdeckelement 66 ist dazu vorgesehen, eine Lüftung in einem unteren Belüftungsbereich zu ermöglichen. Das weitere Abdeckelement 68 ist dazu vorgesehen, eine Lüftung in einem oberen Belüftungsbereich zu ermöglichen. Das Abdeckelement 66 und/oder das weitere Abdeckelement 68 könnte mehrteilig ausgebildet sein. Die Brillenvorrichtung 10 weist ein Dichtelement 70 auf. Das Dichtelement 70 ist aus einem weichen, reversibel verformbaren, Werkstoff, wie beispielsweise einem Schaumstoff, ausgebildet. Das Dichtelement 70 ist dazu vorgesehen, die Brillenvorrichtung 10 fluiddicht mit einem Gesicht des Benutzers zu verbinden.

Die Brillenvorrichtung 10 weist eine Lichtsensoreinheit 16 auf. Die Lichtsensoreinheit 16 ist oberhalb der Durchsichteinheit 12 angeordnet. Alternativ könnte die Lichtsensoreinheit 16 auch seitlich und/oder unterhalb der Durchsichteinheit 12 angeordnet sein. Die Lichtsensoreinheit 16 ist außerhalb des Sichtfelds von dem Benutzer angeordnet. Die Lichtsensoreinheit 16 ist an dem Rahmen 32 angeordnet. Alternativ könnte die Lichtsensoreinheit 16 an dem Brillenbügel 34 und/oder an einem Brillenband und/oder an dem Helm und/oder an einem Visier angeordnet sein. Die Lichtsensoreinheit 16 ist in den Rahmen 32 von der Brillenvorrichtung 10 und/oder in den Brillenbügel 34 von der Brillenvorrichtung 10 integriert. Es ist denkbar, dass die Lichtsensoreinheit 16 an den Rahmen 32 von der Brillenvorrichtung 10 und/oder an den Brillenbügel 34 von der Brillenvorrichtung 10 montiert ist.

Die Lichtsensoreinheit 16 weist eine Solarzelle 28 und eine Fotodiode 30 auf. Die Lichtsensoreinheit 16 könnte auch nur eine Solarzelle 28 oder nur eine Fotodiode 30 aufweisen. Die Lichtsensoreinheit 16 könnte mehrere Solarzellen 28 aufweisen. Die Lichtsensoreinheit 16 könnte mehrere Fotodioden 30 aufweisen. Die Lichtsensoreinheit 16 bildet sechs Messbereiche 22, 26 aus. Die Lichtsensoreinheit 16 könnte auch eine von sechs verschiedene Anzahl an Messbereichen 22 aufweisen. Die Lichtsensoreinheit 16 ist dazu vorgesehen, auf die Messbereiche 22, 26 einfallendes Licht in eine elektrische Spannung zu wandeln. Die elektrische Spannung ist proportional zu der Lichtintensität. Die Lichtsensoreinheit 16 ist dazu vorgesehen, ein von einer Umgebungshelligkeit abhängiges Sensorsignal zu erzeugen. Das Sensorsignal ist als eine elektrische Spannung gebildet. Das Sensorsignal ist Basis für einen automatisch eingestellten Grad einer variablen Verdunkelung des Verdunkelungselements 14.

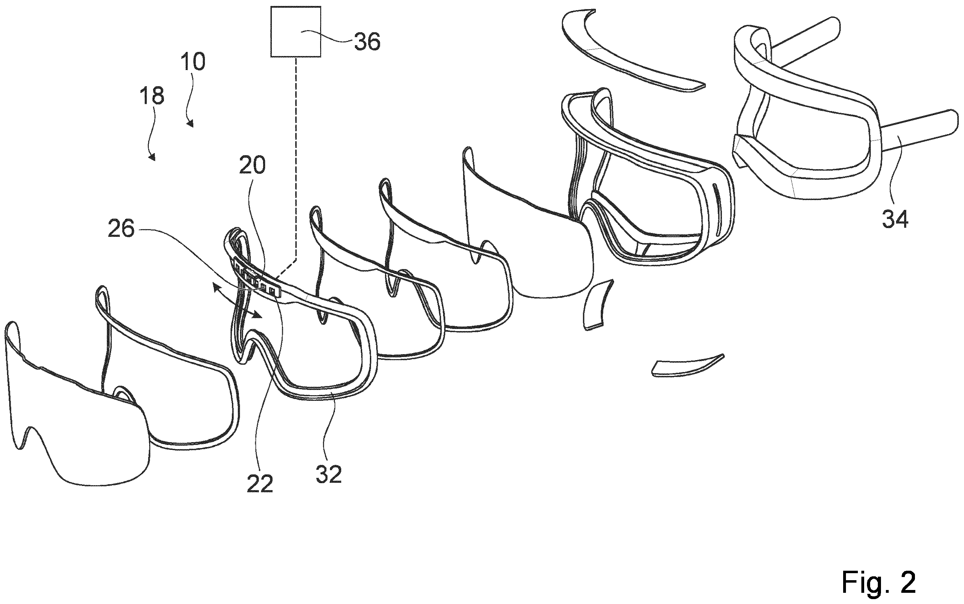

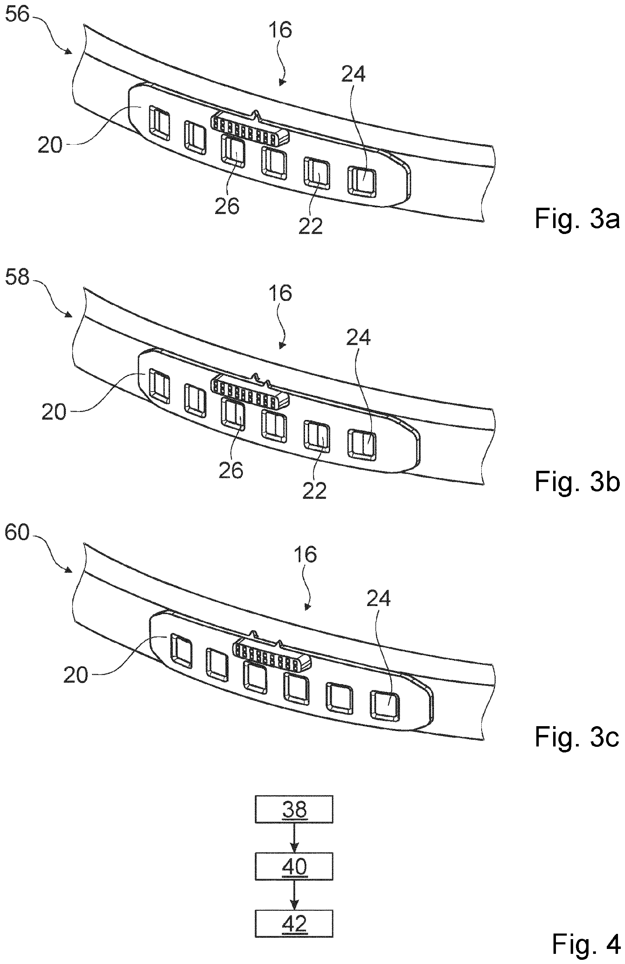

Die Figur 2 zeigt die Brillenvorrichtung 10. Die Brillenvorrichtung 10 weist eine Abschalteinheit 18 auf. Die Abschalteinheit 18 stellt eine manuell bedienbare Abschaltfunktion für das Sensorsignal der Lichtsensoreinheit 16 bereit. Die Abschalteinheit 18 weist ein mechanisches Abdeckelement 20 auf. Das mechanische Abdeckelement 20 ist in einem Betriebszustand 60 (vgl. Fig. 3c) dazu vorgesehen, alle Messbereiche 22, 26 der Lichtsensoreinheit 16 vollständig abzudecken. Das mechanische Abdeckelement 20 ist in einem weiteren Betriebszustand 58 (vgl. Fig. 3b) dazu vorgesehen, die Messbereiche 22, 26 der Lichtsensoreinheit 16 teilweise abzudecken. Das mechanische Abdeckelement 20 ist stufenlos einstellbar. Das stufenlos einstellbare mechanische Abdeckelement 20 ermöglicht eine stufenlose Regelung des Verdunkelungselements 14. Das mechanische Abdeckelement 20 ist als ein Schieber ausgebildet. Das mechanische Abdeckelement 20 ist entlang einer Längserstreckung von der Durchsichteinheit 12 abschnittsweise beweglich. Das mechanische Abdeckelement 20 ist abschnittsweise rotationsbeweglich um einen Rotationsmittelpunkt von einem konkav gebogenen Verlauf der Durchsichteinheit 12 gelagert. Alternativ könnte der Schieber auch durch eine Drehbewegung oder eine Klappbewegung oder eine vergleichbare Bewegung die Messbereiche 22, 26 abdecken. Die Abschalteinheit 18 ist alternativ oder zusätzlich mittels einer Fernsteuerung 36 bedienbar. Die Fernsteuerung 36 ist als ein Mobilgerät ausgebildet. Das Mobilgerät könnte ein Smartphone oder ein Tablet oder ein vergleichbares Mobilgerät sein.

Das mechanische Abdeckelement 20 ist an dem Rahmen 32 und/oder an dem Brillenbügel 34 angeordnet. Das mechanische Abdeckelement 20 ist in den Rahmen 32 und/oder in den Brillenbügel 34 integriert. Es ist denkbar, dass das mechanische Abdeckelement 20 an den Rahmen 32 und an den Brillenbügel 34 montiert ist. Das mechanische Abdeckelement 20 ist formschlüssig mit dem Rahmen 32 der Brillenvorrichtung 10 und/oder dem Brillenbügel 34 verbunden.

Die Figuren 3a bis 3c zeigen das mechanische Abdeckelement 20. Das mechanische Abdeckelement 20 ist aus einem opaken Kunststoff ausgebildet. Das mechanische Abdeckelement 20 weist sechs Durchgangsöffnungen 24 auf. Das mechanische Abdeckelement 20 könnte eine von sechs abweichende Anzahl an Durchgangsöffnungen 24 aufweisen. Das mechanische Abdeckelement 20 weist gleich viele Durchgangsöffnungen 24 auf, wie die Lichtsensoreinheit 16 Messbereiche 22, 26 umfasst. Die Durchgangsöffnungen 24 sind rechteckförmig ausgebildet. Alternativ könnten die Durchgangsöffnungen 24 auch rund oder dreieckförmig oder andersförmig ausgebildet sein. Die Durchgangsöffnungen 24 sind vollständig in Umfangsrichtung von dem mechanischen Abdeckelement 20 umrandet. Die Durchgangsöffnungen 24 sind dazu vorgesehen, in einem weiteren Betriebszustand 56 (vgl. Fig. 3a) vollständig mit den Messbereichen 22, 26 der Lichtsensoreinheit 16 zu überlappen. Es ist denkbar, dass die Durchgangsöffnungen 24 mit einem durchsichtigen oder teildurchsichtigen optischen Element fluiddicht verschlossen sind. Das optische Element könnte wasserabweisend beschichtet sein, um eine Beeinträchtigung der Funktion oder eine Beschädigung der Lichtsensoreinheit 16 durch Regentropfen zu verhindern. Beispielsweise könnte das teildurchsichtige optische Element ausschließlich für bestimmte Wellenlängen elektromagnetischer Strahlung durchlässig sein. Beispielsweise könnte das teildurchsichtige optische Element nur für UV-Strahlung durchlässig sein, um das menschliche Auge vor UV-Strahlung zu schützen.

Die Figur 3a zeigt den Betriebszustand 56. Die Durchgangsöffnungen 24 des mechanischen Abdeckelements 20 sind in dem Betriebszustand 56 vollständig mit den Messbereichen 22, 26 überlappend angeordnet. Der Betriebszustand 56 bei vollständig mit den Messbereichen 22, 26 überlappenden Durchgangsöffnungen 24 ist dazu vorgesehen, einen automatisch eingestellten Grad der variablen Verdunkelung des Verdunkelungselements 14 mit einer maximalen Verdunkelung zu erreichen.

Die Figur 3b zeigt den Betriebszustand 58. Die Durchgangsöffnungen 24 des mechanischen Abdeckelements 20 sind in dem Betriebszustand 58 teilweise mit den Messbereichen 22, 26 überlappend angeordnet. Der Betriebszustand 58 bei teilweise mit den Messbereichen 22, 26 überlappenden Durchgangsöffnungen 24 ist dazu vorgesehen, einen automatisch eingestellten Grad der variablen Verdunkelung des Verdunkelungselements 14 mit einer benutzerabhängigen Verdunkelung zu erreichen.

Die Figur 3c zeigt den Betriebszustand 60. Die Durchgangsöffnungen 24 des mechanischen Abdeckelements 20 sind in dem Betriebszustand 60 überlappungsfrei mit allen Messbereichen 22, 26 der Lichtsensoreinheit 16 angeordnet. Der Betriebszustand 60 bei überlappungsfrei mit den Messbereichen 22, 26 angeordneten Durchgangsöffnungen 24 ist dazu vorgesehen, eine automatische Verdunkelung des Verdunkelungselements 14 vollständig abzuschalten.

Die Figur 4 zeigt ein schematisches Ablaufdiagramm eines Verfahrens zu einem Betrieb einer Brillenvorrichtung 10.

In einem Verfahrensschritt 38 werden die Durchgangsöffnungen 24 von dem mechanischen Abdeckelement 20 vollständig überlappend mit den Messbereichen 22, 26 ausgerichtet. Die von den Messbereichen 22, 26 erfasste Umgebungshelligkeit wird von der Solarzelle 28 und/oder der Fotodiode 30 in das elektrisches Spannungssignal umgewandelt. Das Verdunkelungselement 14 wird durch das elektrische Spannungssignal, abhängig von der Umgebungshelligkeit, verdunkelt. Die Durchsichteinheit 12 wird teilweise automatisiert und variabel verdunkelt.

In einem Verfahrensschritt 40 wird die Durchsichteinheit 12 teilweise verdunkelt. Zu der teilweisen Verdunkelung der Durchsichteinheit 12 wird das mechanische Abdeckelement 20 teilweise über die Messbereiche 22, 26 geschoben. Durch das teilweise Abdecken der Messbereiche 22, 26 durch das mechanische Abdeckelement 20 wird eine Grundhelligkeit der Durchsichteinheit 12 durch den Bediener voreingestellt. Die Durchsichteinheit 12 wird teilweise automatisiert und variabel verdunkelt. Die Verdunkelung basiert auf einem von einer Umgebungshelligkeit abhängigen Sensorsignal. Zu der Verdunkelung von der Durchsichteinheit 12 wird das Verdunkelungselement 14 verdunkelt. Das Sensorsignal wird als Basis für einen Grad der Verdunkelung herangezogen.

In einem Abschaltschritt 42 wird das Sensorsignal über eine bedienbare Abschalteinheit 18 abgeschaltet. Durch die Abschaltung von dem Sensorsignal wird die Verdunkelung von der Durchsichteinheit 12 abgeschaltet. Das Sensorsignal wird durch ein mechanisches Abdecken einer das Sensorsignal erzeugenden Lichtsensoreinheit 16 abgeschaltet. Das Sensorsignal wird in dem Abschaltschritt 42 durch das mechanische Abdeckelement 20 abgedeckt. Das mechanische Abdeckelement 20 wird zu dem mechanischen Abdecken über die Messbereiche 22, 26 von der Lichtsensoreinheit 16 geschoben. Alternativ kann das Sensorsignal über die Abschalteinheit 18 mit einem Mobilgerät abgeschaltet werden.

### Bezugszeichen

- 10: Brillenvorrichtung
- 12: Durchsichteinheit
- 14: Verdunkelungselement
- 16: Lichtsensoreinheit
- 18: Abschalteinheit
- 20: Abdeckelement
- 22: Messbereich
- 24: Durchgangsöffnung
- 26: Messbereich (weitere)
- 28: Solarzelle
- 30: Fotodiode
- 32: Rahmen
- 34: Brillenbügel
- 36: Fernsteuerung
- 38: Verfahrensschritt
- 40: Verfahrensschritt
- 42: Abschaltschritt
- 44: Brille
- 46: Teilbereich
- 48: Scheibe
- 50: Scheibe
- 54: Klebeelement
- 56: Betriebszustand
- 58: Betriebszustand
- 60: Betriebszustand
- 62: Klebeelement
- 64: Rahmen
- 66: Abdeckelement
- 68: Abdeckelement
- 70: Dichtelement

## Patentansprüche

1. Brillenvorrichtung (10), insbesondere Skibrille, Sonnenbrille, Motorradbrille oder dergleichen, mit einer Durchsichteinheit (12), welche zumindest ein Verdunkelungselement (14), insbesondere eine Flüssigkristallzelle, aufweist, das zu einer Verdunkelung zumindest eines Teilbereichs (46) der Durchsichteinheit (12) vorgesehen ist, und mit einer Lichtsensoreinheit (16), welche dazu vorgesehen ist, ein von einer Umgebungshelligkeit abhängiges Sensorsignal zu erzeugen, das Basis für einen, insbesondere automatisch eingestellten, Grad einer, insbesondere variablen, Verdunkelung des Verdunkelungselements (14) ist, **gekennzeichnet durch** eine Abschalteinheit (18), welche zumindest eine, insbesondere manuell bedienbare, Abschaltfunktion für das Sensorsignal der Lichtsensoreinheit (16) bereitstellt.

2. Brillenvorrichtung (10) nach Anspruch 1, **dadurch gekennzeichnet, dass** die Abschalteinheit (18) ein mechanisches Abdeckelement (20) aufweist, welches in zumindest einem Betriebszustand (60) dazu vorgesehen ist, zumindest alle Messbereiche (22) der Lichtsensoreinheit (16) zumindest im Wesentlichen vollständig abzudecken.

3. Brillenvorrichtung (10) nach Anspruch 2, **dadurch gekennzeichnet, dass** das mechanische Abdeckelement (20) in zumindest einem weiteren Betriebszustand (58) dazu vorgesehen ist, die Messbereiche (22) der Lichtsensoreinheit (16) lediglich teilweise abzudecken.

4. Brillenvorrichtung (10) nach Anspruch 2 oder 3, **dadurch gekennzeichnet, dass** das mechanische Abdeckelement (20) zumindest eine, vorzugsweise vollständig in Umfangsrichtung von dem Abdeckelement (20) umrandete, Durchgangsöffnung (24) aufweist, welche dazu vorgesehen ist, in zumindest einem weiteren Betriebszustand (56) vollständig mit zumindest einem Messbereich (22) der Lichtsensoreinheit (16) zu überlappen.

5. Brillenvorrichtung (10) nach den Ansprüchen 2 und 4, **dadurch gekennzeichnet, dass** die Durchgangsöffnung (24) des mechanischen Abdeckelements (20) in dem Betriebszustand (60) überlappungsfrei mit dem Messbereich (22), und insbesondere mit allen weiteren Messbereichen (26) der Lichtsensoreinheit (16), ist.

6. Brillenvorrichtung (10) nach einem der vorhergehenden Ansprüche, **dadurch gekennzeichnet, dass** die Lichtsensoreinheit (16) zumindest eine Solarzelle (28) und/oder zumindest eine Fotodiode (30) aufweist.

7. Brillenvorrichtung (10) nach einem der vorhergehenden Ansprüche, **dadurch gekennzeichnet, dass** das mechanische Abdeckelement (20) an einem Rahmen (32) und/oder an einem Brillenbügel (34), insbesondere integriert oder montiert, angeordnet ist.

8. Brillenvorrichtung (10) nach einem der vorhergehenden Ansprüche, **dadurch gekennzeichnet, dass** die Abschalteinheit (18) mittels einer Fernsteuerung (36), insbesondere einem Mobilgerät, wie z.B. einem Smartphone, bedienbar ist.

9. Brille (44) mit einer Brillenvorrichtung (10) nach einem der vorhergehenden Ansprüche.

10. Verfahren zu einem Betrieb einer Brillenvorrichtung (10), insbesondere nach einem der Ansprüche 1 bis 8, mit einer Durchsichteinheit (12), welche in zumindest einem Verfahrensschritt (40) zumindest teilweise, vorzugsweise automatisiert und variabel, verdunkelt wird, wobei die Verdunkelung auf einem von einer Umgebungshelligkeit abhängigen Sensorsignal, welches Basis für einen Grad der Verdunkelung ist, basiert, **dadurch gekennzeichnet, dass** in zumindest einem Abschaltschritt (42) das Sensorsignal über eine, insbesondere manuell bedienbare, Abschalteinheit (18) abgeschaltet wird.

11. Verfahren nach Anspruch 10, **dadurch gekennzeichnet, dass** in dem Abschaltschritt (42) das Sensorsignal durch ein mechanisches Abdecken einer das Sensorsignal erzeugenden Lichtsensoreinheit (16) abgeschaltet wird.
